# EUROPEAN PATENT APPLICATION

(11) **EP 4 388 979 A1**
(43) Date of publication of application: **26.06.2024**
(21) Application number: 22215001.3
(22) Date of filing: 20.12.2022
(51) Int. Cl.: A61B 5/00, A61B 5/257

(54) **AGGREGATE COMPRISING A VITAL SIGN MONITORING SENSOR BODY**

(71) Applicant: Novosense AB, 223 63 Lund (SE)
(72) Inventor: Sebelius, Fredrik, 247 34 Södra Sandby (SE); Tilly, Jonas, 234 40 Lomma (SE)
(74) Representative: AWA Sweden AB

(57) **Abstract**

An aggregate (1) is provided which comprises: a vital sign monitoring sensor body (3) comprising at least one sensor and a carrier (2) for supporting the vital sign monitoring sensor body (3). The carrier (2) comprises a supporting member (8; 10) configured to support the vital sign monitoring sensor body (3) on the carrier (2). The aggregate further comprises a connecting member (14) comprising a first connecting surface (16a) and a thereto opposed second connecting surface (18a), wherein the first connecting surface (16a) connects to an attachment surface (24) of the vital sign monitoring sensor body (3) and the second connecting surface (18a) is configured to adhesively adhere to a patient's skin.

## Description

### Technical field

The present invention relates to an aggregate comprising of a vital sign monitoring sensor body.

### Background

A cardiac cycle can be described as the activation of certain specialized heart conduction cells in a predictable sequence, which leads to a coordinated and sequential contradiction of the arterial and ventricular muscle fibers. The electrical signal associated with the muscle action is transmitted through various tissues and ultimately reaches the surface of the body where it can be measured. Such a measurement is called ECG, which stands for electrocardiogram. Electrical equipment for such measurements is used for monitoring and/or recording ECG data. The equipment may be stationary or portable.

The stationary ECG equipment is electrical monitoring and recording devices, i.e. vital sign monitoring sensor bodies, which are connected to a patient by wires. In current use, such equipment utilizes surface electrodes located on the body of the patient and connected by wires to an electrocardiograph machine which allows the detected heart signals to be displayed.

The portable ECG equipment can be divided into recorders and transmitters. In both cases wires from multiple electrodes applied to the body of the patient are connected to a recorder or transmitter unit, hung around the patient's neck. The recording unit is a self-contained unit such that the patient may move around. The transmitter unit further contains some sort of radio equipment, which makes it possible for the patient to move around while still being monitored by a stationary unit receiving the measuring data signals from the transmitter unit.

WO07094729 discloses a system and method for wireless generation of ECG leads. The system consists of at least one mobile ECG sensing unit to be mounted by an adhesive to the patient's body and an ECG receiving unit which is a stationary radio unit. The ECG receiver unit comprises a radio module with transmitting and receiving capability, a computation module capable of processing and synthesizing ECG signals, and a communication module for communicating with other standard ECG systems. The ECG sensing units, typically three, cooperate during an initial phase of the operation of the system with a connection unit. During this initial phase, each sensing unit is connected by a multi cable connection to the connection unit which is also connected by a single cable connection to each one of a plurality of passive sensing units. The cable connections and the connection unit are initially interconnected for the recording of standard ECG signals simultaneously with local bipolar signals. After a shorter recording session, lasting seconds to minutes, the system is calibrated. After calibration, the connection unit with its cable connections and the passive electrodes are removed. Remaining on the body are the three ECG sensing units. Accordingly, after calibration this system is wireless. The ECG sensing units are applied by adhesive to the patient's body. To protect the adhesive until application of the sensing units to the patient's body, a release strip is provided which has to be removed before application of the sensing body to the skin. Further, to facilitate the mounting of the system, the ECG sensing units may be provided pre-mounted to the necessary cables. The provision of the release strips and also the pre-mounted cables do facilitate and speed up the application of the sensing units to the body. Also, it reduces the failure rate caused by incorrect connection of the cables. Still, there is room for an even more simplified mounting of the sensing units to the patient's body with an even lower failure rate.

### Summary of the invention

In view of the above, it is an object of the present invention to provide an aggregate comprising a vital sign monitoring sensor body that in a very simple manner may be directly applied to the patient's body.

Further, another object is that the aggregate should allow for a safe storing and handling of the vital sign monitoring sensor body, including any pre-connected cables until application to the patient's body.

According to a first aspect the invention relates to an aggregate comprising:
a vital sign monitoring sensor body comprising at least one sensor;
a carrier for supporting the vital sign monitoring sensor body, the carrier comprising a supporting member configured to support the vital sign monitoring sensor body on the carrier;
a connecting member comprising a first connecting surface and a thereto opposed second connecting surface, wherein the first connecting surface connects to an attachment surface of the vital sign monitoring sensor body and the second connecting surface is configured to adhesively adhere to a patient's skin.

Accordingly, an aggregate is provided wherein the vital sign monitoring sensor body may be removed from the carrier and be adhesively adhered to the patient's skin by means of the second connecting surface of the connecting member. While being supported on the carrier, the sensor body may be removably connected to the supporting member of the carrier by means of the second connecting surface of the connecting member. Thereby, as the operator removes the vital sign monitoring sensor body from the carrier by gripping and lifting the sensor body, the connecting member remains attached to the vital sign monitoring sensor body with the now freely exposed second adhesive surface of the connecting member. The vital sign monitoring sensor body may be directly attached to the patient's body by pressing the freely exposed second adhesive surface against the patient's body. Thus, there is no release strip that must be removed, which is often experienced as difficult when using gloves. Further, the operator may handle the vital sign monitoring sensor body with one hand while holding the carrier with his/her other hand.

Further, since the vital sign monitoring sensor body may be removably connected to the supporting member of the carrier until the moment when the operator actively removes the vital sign monitoring sensor body, the vital sign monitoring sensor body may be safely protected during storing, transportation and handling of the aggregate. Thus, the second connecting surface may serve a dual purpose - primarily as an attachment means to the body of the patient and optionally, and secondary as a safety arrangement that secures the sensor body to the carrier during handling/transportation of the aggregate.

The vital sign monitoring sensor body may comprise a communication module. The communication module may be a radio module configured to communicate with a receiver. In another embodiment, the communication module may be a memory, such as a flash memory. In the event of a memory, registered data can be stored locally for later analysis. As long as the aggregate and its contents are not to be used, which is the case during transportation and general handling/storing of the aggregate, it is preferred that the vital sign monitoring sensor body is set to a passive operation mode. This means that any communication module forming part of the sensor body also should be inoperable. This saves battery time and also reduces any noise signals.

The vital sign monitoring sensor body may be configured to be set from a passive operating mode to an active operating mode upon removal of the sensor body from the carrier by breaking of a magnetic field being formed between a magnetic sensor of the vital sign monitoring sensor body and a magnet of the carrier. Said magnetic field is formed as long as the vital sign monitoring sensor body is supported by the carrier. The magnetic sensor may be a Reed switch.

The vital sign monitoring sensor body may comprise an electrical switch, and the vital sign monitoring sensor body may be configured to be set from a passive operating mode to an active operating mode by removing an insulating member being arranged in operational contact with the electrical switch in a condition when the vital sign monitoring sensor body is supported on the carrier.

The insulating member is upon delivery of the aggregated arranged in operational contact with the electrical switch, thereby breaking any communication across the switch. To set the vital sign monitoring sensor body from the passive operating mode to the active operating mode, the insulating member must be removed, thereby allowing a communication across the switch.

The insulating member may be connected to the carrier, such as to the supporting member. Thereby, the electrical switch may be configured to be automatically set from the passive operating mode to the active operating mode when removing the vital sign monitoring sensor body from the carrier. The insulating member may be a protrusion supported by the carrier

In another embodiment, the insulating member, such as an insulating distance or insulating film may be supported by the vital sign monitoring sensing body, separate from the carrier, whereby the insulating member is configured to be manually removed to thereby set the switch from the passive operating mode to the active operating mode.

The vital sign monitoring sensor body may be mechanically and releasably supported by the supporting member of the carrier. In a condition when the vital sign monitoring sensor body is mechanically supported by the carrier, there may be no, or substantially no, adhesive contact between the supporting member of the carrier and the second connecting surface of the connecting member.

The first connecting surface may in one embodiment mechanically connect the connecting member to the attachment surface of the vital sign monitoring sensor body by at least one mechanical fastening member. The at least one mechanical fastening member may by way of example be a clip, a rivet or a screw.

The first connecting surface may in one embodiment adhesively connect the connecting member to the attachment surface of the vital sign monitoring sensor body by a releasable adhesive or by a permanent adhesive.

The vital sign monitoring sensor body may be adhesively and releasably supported by the supporting member of the carrier by an adhesive of the second connecting member. Thus, the adhesive of the second connecting member may serve the dual purpose of removably supporting the vital sign monitoring sensor body to the carrier and also allowing the vital sign monitoring sensor body to be attached to the patient's skin.

At least a portion of the supporting member of the carrier may be coated with an easy release coating material.

A contact area formed between the supporting member of the carrier and the second connecting surface of the connecting member may be smaller than a total cross-sectional area of the second connecting surface of the connecting member. The difference in contact area contributes to an easy removal of the vital sign monitoring sensor body from the carrier.

An adhesive strength between the attachment surface of the vital sign monitoring sensor body and the first connecting surface of the connecting member may be lower than the adhesive strength between a patient's skin and the second connecting surface of the connecting member. Accordingly, as the operator lifts the vital sign monitoring sensor body in a direction away from the patient's body, the connecting member will remain attached to the patient's body. The vital sign monitoring sensor body will then after removal from the patient's body be free of any adhesives. This facilitates future handling of the sensor body during e.g. re-cycling or re-conditioning. Further, it will cause less discomfort or pain to the patient when removing the adhesive layer alone from the patient's skin.

It is preferred that the adhesive strength between the attachment surface of the vital sign monitoring sensor body and the first connecting surface of the connecting member is stronger than the adhesive strength between the supporting member of the carrier and the second connecting surface of the connecting member. Accordingly, as the operator lifts the vital sign monitoring sensor body from the carrier, the connecting member will follow the vital sign monitoring sensor body.

The connecting member may comprise a skirt portion extending past an outer boundary of the attachment surface of the vital sign monitoring sensor body, the skirt portion of the connecting member being more flexible than the vital sign monitoring sensor body. The flexible skirt increases the connecting strength between the patient's skin and the connecting member as seen in a condition when the sensor body is mounted to the patient's body. Also, the flexible skirt allows a better conformation to the patient's body than the relatively more rigid sensor body.
By the increased strength between the connecting member and the patient's skin and the better conformation of the skirt to the patient's body, the sensor body will separate from the connecting member while the connecting member remains adhered to the skin. The thus adhesive-free sensor body is more easy to recycle or recondition. Also, the connecting member can be peeled off separately from the skin in a less painful manner.

In another embodiment, the vital sign monitoring sensor body may comprise a flange portion, and the connecting member comprise a skirt portion extending past an outer boundary of the flange portion of the vital sign monitoring sensor body, said skirt portion of the connecting member being more flexible than the flange portion of the vital sign monitoring sensor body. The flange portion of the vital sign monitoring sensor body may in turn be more flexible than the vital sign monitoring sensor body. The flange portion extends in the lateral direction away from the housing of the vital sign monitoring sensor body. The flange portion may be integrally formed with the housing of the vital sign monitoring sensor body or be mounted thereto.

The combination of the flexible skirt portion and the flexible flange portion further increases the connecting strength between the patient's skin and the connecting member as seen in a condition when the sensor body is mounted to the patient's body. Also, the solution allows an even better conformation to the patient's body than the relatively more rigid sensor body. The flange portion of the sensor body should be flexible enough to allow the flange portion to be pivoted in view of the housing of the sensor body.

The vital sign monitoring sensor body may comprise one or more sensors from the group consisting of an ECG (Electrocardiography) sensor, a pulse oximeter sensor, a blood pressure sensor, a temperature sensor, a glucose sensor, a cortisol sensor, an oxygen sensor, a carbon dioxide sensor and a respiration sensor.
The skilled person realizes that the vital sign monitoring sensor body may also comprise other types of sensors.

The aggregate may comprise two or more vital sign monitoring sensor bodies, and the two or more sensor bodies may be connected by one or more electrically conducting members while being supported in the carrier, and the connection with the one or more electrically conducting members may be configured to remain after removal of the two or more vital sign monitoring sensor bodies from the carrier. Alternatively, the one or more electrically conducting members may be configured to be disconnected after removal of the two or more vital sign monitoring sensor bodies from the carrier.

The electrically conductive members may be formed by a laminate comprising a flexible film supporting one or more flexible insulators and one or more flexible metal films. The one or more flexible metal films may have a pattern that makes it possible to electrically connect multiple electrical signals. The pattern may be a printed pattern.

The aggregate may comprise an electrical connector arranged in direct or indirect communication with the vital sign monitoring sensor body, said electrical connector allowing the vital sign monitoring sensor body to be charged when being supported by the carrier. This allows a prolonged shelf life of the aggregate. In yet another embodiment, the vital sign monitoring sensor body may comprise a rechargeable battery configured to be wirelessly charged while being supported by the carrier.

The vital sign monitoring sensor body may be provided with hydrogel on a surface thereof that is configured to face a patient's skin when the vital sign monitoring sensor body is used on a patient. The purpose of the hydrogel is to improve connectivity between the vital sign monitoring sensor body and the patient's skin. To avoid the hydrogel from drying out over time, the aggregate may further comprise a moist carrier, such as a moist sponge. To retain the humidity until use, the carrier may be provided with a protective sealing film that is sealed to the carrier and that is to be removed by the operator. The protective film further ensures a sterile environment inside the thus sealed-off carrier.

The hydrogel may be arranged on one or more isolated areas on the attachment surface of the vital sign monitoring sensor body. The connecting member may be provided with corresponding cut-outs, thereby preventing contact between the hydrogel and the connecting member and also between the hydrogel and the carrier.

A further scope of applicability of the present invention will become apparent from the detailed description below. However, it should be understood that the detailed description and specific examples, while indicating preferred embodiments of the invention, are given by way of illustration only, since various changes and modifications within the scope of the invention will become apparent to those skilled in the art from this detailed description.

Hence, it is to be understood that this invention is not limited to the particular component parts of the aggregate described as such component parts may vary. It is also to be understood that the terminology used herein is for the purpose of describing particular embodiments only, and is not intended to be limiting. It must be noted that, as used in the specification and the appended claim, the articles "a," "an," "the," and "said" are intended to mean that there are one or more of the elements unless the context clearly dictates otherwise. Thus, for example, reference to "a unit" or "the unit" may include several devices, and the like. Furthermore, the words "comprising", "including", "containing" and similar wordings does not exclude other elements or steps.

### Brief Description of the Drawings

The above and other aspects of the present invention will now be described in more detail, with reference to appended drawings showing embodiments of the invention. The figures should not be considered limiting the invention to the specific embodiment; instead they are used for explaining and understanding the invention.

As illustrated in the figures, the sizes of layers and regions are exaggerated for illustrative purposes and, thus, are provided to illustrate the general structures of embodiments of the present invention. Like reference numerals refer to like elements throughout.
Fig. 1 illustrates an example of an aggregate in line with the present invention with three vital sign monitoring sensor bodies.
Fig. 2 discloses the aggregate of Fig. 1 as seen from below.
Fig. 3 discloses one embodiment of the attachment member.
Fig. 4 schematically discloses one embodiment of a vital sign monitoring sensor body with a connecting member adhesively connecting thereto.
Fig. 5 discloses one embodiment of a vital sign monitoring sensor body with a connecting member where the connecting member mechanically connects to the sensor body.
Fig. 6 discloses one embodiment of a vital sign monitoring sensor body using magnet activation.
Figs. 7a and 7b disclose two embodiments of a vital sign monitoring sensor body using an electrical switch.
Fig. 8a-8c disclose one embodiment where the connecting member comprises a skirt portion.
Fig. 9a-9d disclose one embodiment where the vital sign monitoring sensor body comprises a flange portion and the connecting member comprises a skirt portion.
Fig. 10 discloses an embodiment of the aggregate where the vital sign monitoring sensor body is supported by the carrier.

### Detailed description

The present invention will now be described more fully hereinafter with reference to the accompanying drawings, in which currently preferred embodiments of the invention are shown. This invention may, however, be embodied in many different forms and should not be construed as being limited to the embodiments set forth herein. Rather, these embodiments are provided for thoroughness and completeness, and to fully convey the scope of the invention to the skilled person.

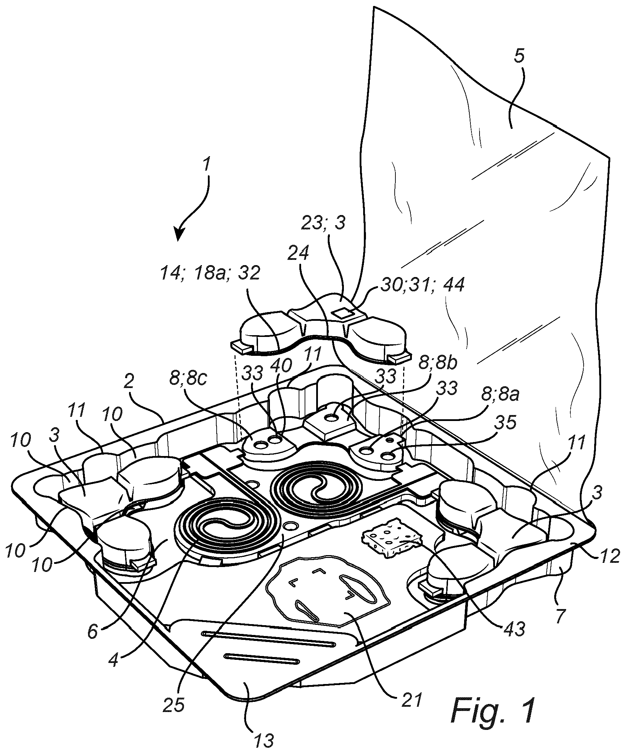

Now turning to Fig. 1 one embodiment of the aggregate 1 with its parts will be described. The aggregate 1 comprises a carrier 2 that supports three vital sign monitoring sensor bodies 3, in the following interchangeably referred to as sensor bodies 3. One of the sensor bodies 3 is for illustrative reasons disclosed in a lifted position. Although three sensor bodies 3 are disclosed, it is to be stressed that the aggregate 1 may comprise at least one sensor body 3.

The sensor body 3 comprises a housing 23 which encapsulates one or more, highly schematically disclosed sensors 30 from the group consisting of an ECG (Electrocardiography) sensor, a pulse oximeter sensor, a blood pressure sensor, a temperature sensor, a glucose sensor, a cortisol sensor, an oxygen sensor, a carbon dioxide sensor and a respiration sensor. The skilled person realizes that the vital sign monitoring sensor body 3 also may comprise other types of sensors 30.

The housing 23 further encapsulates at least one communication module 31. The at least one communication module 31 which is highly schematically disclosed may be a radio module configured to communicate with a receiver. In another embodiment, the communication module 31 may be a memory, such as a flash memory. In the event of a memory, registered data can be stored locally for later analysis. Further, the housing encapsulates a PCB (printed circuit board) 44 to which the one or more sensor 30 and the communication module 31 is connected.

The housing 23 may be formed by a silicone-based material to allow easy re-conditioning of used sensor bodies 3.

The vital sign monitoring sensor body may comprise an optional flange portion 32 to be further discussed below. The flange portion 32 may be integrally formed with the housing 23 or be mounted thereto. The flange portion 32 must not have a continuous extension. The flange portion 32 and the housing 23 may be formed by the same material or by different materials.

The vital sign monitoring sensor body 3 may be a single-use sensor body. Alternatively, the vital sign monitoring sensor body 3 may be a reusable sensor body.

The lower side of the vital sign monitoring sensor body 3 forms an attachment surface 24 which during use is intended to be arranged facing a patient's body.

The three sensing bodies 3 are disclosed as being connected by one or more electrically conducting members 4 while being supported in the carrier 2. The connection with the one or more electrically conducting members 4 may be configured to remain after removal of the sensor bodies 3 from the carrier 2. Alternatively, the one or more electrically conducting members 4 may be configured to be disconnected after removal of the sensor bodies 3 from the carrier 2. The conducting members 4 may in their easiest form be constituted by wires. Alternatively, the electrically conductive members 4 may be formed by a laminate comprising a flexible film supporting one or more flexible insulators and one or more flexible metal conductors. The one or more flexible metal conductors may have a pattern that makes it possible to electrically connect multiple electrical signals. The pattern may be a printed pattern.

The conducting members 4 may be used to calibrate the sensor bodies 3 before initiating measurements. The sensor bodies 3 may be pre-connected to the conducting members 4 or be arranged to be connected thereto by the operator during or after mounting of the sensor bodies 3 to a patient's skin.

In the disclosed embodiment, the conducting members 4 are arranged in spirals on a support 25 supported by the carrier 2. The conducting members 4 are arranged to be pulled off from the support 25 as the operator lifts a sensor body 3 that is pre-connected thereto. Hence, as the operator lifts a sensor body 3 from the carrier 2, a corresponding length of the conducting member(s) 4 is released and pulled off from the support 25. The length of the thus formed wiring should be adapted to a typical distance between two sensor bodies 3 attached to a patient's body.

The aggregate 1 may comprise an electrical connector 40 (highly schematically illustrated) arranged in direct or indirect communication with the at least one sensor body 3. The electrical connector 40 is configured to allow the sensor body 3 to be charged when being supported by the carrier 2. In yet another embodiment, the vital sign monitoring sensor body 3 may comprise a rechargeable battery (not illustrated) configured to be wirelessly charged while being supported by the carrier. This kind of wireless charging is well known in the art as Qi, being an open interface standard that defines wireless power transfer using induction.

As long as the aggregate 1 and its contents is not to be used, which is the case during transportation and general handling/storing of the aggregate, it is preferred that the vital sign monitoring sensor body 3 with its sensors 30 and communication module 31 is in a passive operation mode, i.e. not sending out any signals. This saves battery time and also reduces any noise signals.

The carrier 2 is provided as a unitary injection moulded, thin-walled tray with a bottom 6 and a circumferential rim 7. The carrier 2 will, unless nothing else is given, be described as oriented with its bottom 6 extending in a horizontal plane. The bottom 6 is provided with one supporting member 8 per sensor body 3. Each sensor body 3 is supported by a respective supporting member 8. In the disclosed embodiment the supporting member 8 has an overall shape corresponding to the footprint of the vital sign monitoring sensor body 3. Other shapes are possible. The supporting member 8 is disclosed as being formed with three elevated supporting portions 8a-8c on which the vital sign monitoring sensor body 3 is supported. The number and shape of these supporting portions 8a-8c may differ with remained function. As will be described below, a contact area formed between the supporting member 8 of the carrier 2 and a second connecting surface 18 of a connecting member 14 to be described below, may be smaller than the total cross-sectional area of the second connecting surface 18 of the connecting member 14. The difference in contact area contributes to an easy removal of the vital sign monitoring sensor body from the carrier.

The supporting member 8 may be coated with an easy release coating material. The release between an adhesive and the coating material should be in the form of a so-called interfacial fracture, e.g. a debonding occurs between the adhesive and the coating material. Thereby no adhesive residues will remain on the supporting member 8. The coating material depends on the type of adhesive to interact with. By way of example, if an acrylic-based adhesive is used, the coating material may be a silicon-based material. Likewise, if a silicon-based adhesive is used, the coating material may be an acrylic-based material. Other types of coating materials are wax, silicon oil, and talc. The very same property could be provided by a suitable selection of plastics used in the carrier 2.

The bottom 6 of the carrier 2 may be provided with a schematic illustration 21 to the operator on the intended positions of the sensor bodies 3 on a patient's body.

The circumferential rim 7 extends in the vertical direction along the circumferential extension of the bottom 6. The rim 7 is disclosed as comprising optional recesses 11 arranged adjacent each vital sign monitoring sensor body 3. The recesses 11 allow the operator to easily grip and lift a sensor body 3 with two fingers. Also, the recesses 11 contribute to the overall rigidity of the carrier 11.

The upper end of the circumferential rim 7 is provided with an outwardly extending brim 12. The brim 12 serves at least two purposes - providing a gripping surface and providing an attachment surface for a protective sealing film 5. The sealing film 5 may be welded or glued against the top surface of the brim 12. In the disclosed embodiment the brim 12 is provided in one of its corners with a gripping means 13.

The aggregate 1 with its contents is sealed off by the sealing film 5 which is arranged to be pulled off by the operator upon opening of the aggregate 1. In the disclosed embodiment, the sealing film 5 is disclosed as being partly pulled off. By the sealing film 5, the aggregate 1 may be provided as a sterile unit. Also, by the sealing film 5, all adhesive surfaces and also any hydrogel that is applied to the sensor bodies 3 may be remain fresh. The hydrogel will be further discussed below.

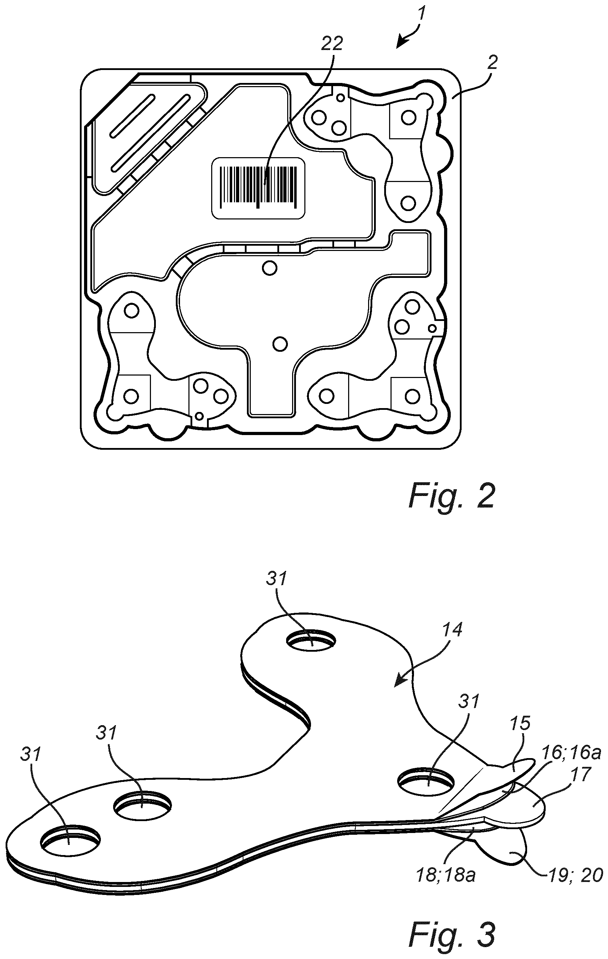

Now turning to Fig. 2 the carrier 2 is seen from below. As a result of the carrier 2 being an injection moulded, thin-walled tray, the underside of the carrier 2 exhibits a shape that directly corresponds to that of the carrier as seen from above. Fig. 2 discloses one example of the underside being used to support identification means 22 in the form of a barcode. The identification means 22 may be used to represent information, such as identification of the sensor bodies 3 and a respective unique radio ID. The identification can also be used to relate the sensor bodies 3 to the identity of the patient.

Fig. 3 discloses one example of a connecting member 14 to be implemented in the aggregate 1. As seen from the top, the connecting member 14 comprises a first release foil 15, a first connecting surface 16 in the form of an adhesive layer 16a, a core layer 17, a second connecting surface 18 in the form of an adhesive layer 18a and a second release foil 19. The first and the second release foils 15, 19 are configured to be removed when assembling the aggregate 1 and do hence not form part of the aggregate 1 as such.

The overall geometry of the connecting member 14 may correspond to the geometry of the attachment surface 24 of the vital sign monitoring sensor body 3.

As will be discussed in embodiments below, the connecting member 14 may be oversized in view of the sensor body 3 to thereby form a skirt portion extending past an outer boundary of the attachment surface 24 of the sensor body 3.

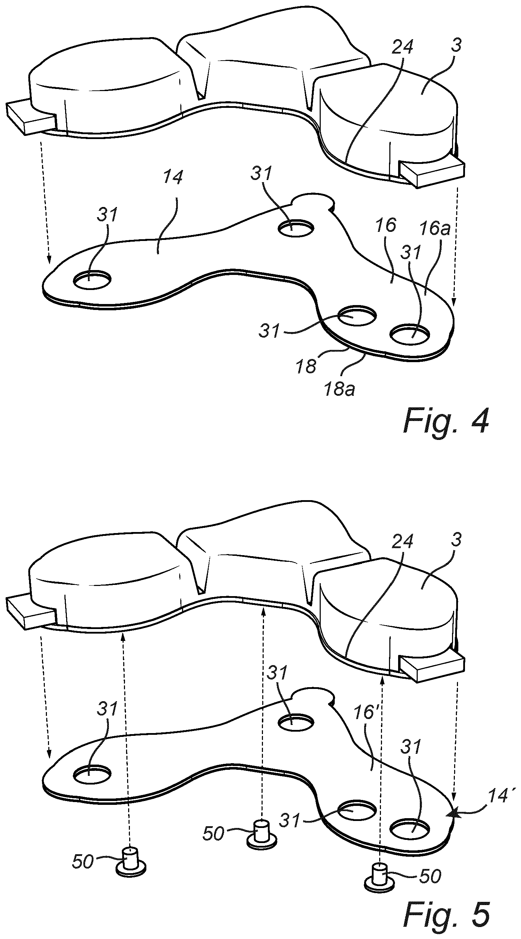

The first connecting surface 16 is adapted to connect the connecting member 14 to the attachment surface 24 of the vital sign monitoring sensor body 3, see Fig. 4. The adhesive layer 16a of the first connecting surface 16 may adhesively connect the connecting member 14 to the attachment surface 24 of the vital sign monitoring sensor body 3 by a releasable adhesive or by a permanent adhesive.

The core layer 17, see Fig. 3, may be formed by e.g. paper or a plastic material and is used as a support for the first and second adhesive layers 16a, 18a. The core layer 17 comprises an optional gripping tab 20 in an edge or corner portion thereof. By the gripping tab 20, the connecting member 14 will be easy to grab and pull off from the patient's skin.

The second connecting surface 18 with the adhesive layer 18a serves two purposes. The first purpose is to prevent the vital sign monitoring sensor body 3 from moving in view of the carrier 2 during handling of the aggregate 1. This is made by the adhesive layer 18a removably adhering to the supporting member 8 of the carrier 2. The second purpose is to allow the vital sign monitoring sensor body 3 to be adhesively attached to the patient's skin. Since a surface of the adhesive layer 18a is freely exposed as the operator lifts the vital sign monitoring sensor body 3 from the carrier 2, the vital sign monitoring sensor body 3 may be moved and attached directly to the patient's skin without the operator having to change his/her grip and especially without being required to remove any release foil from the sensor body 3. During the whole operation the operator can hold the carrier 2 in its gripping means 13 by one hand while mounting the at least one sensor body 3 to the patient's body with the other hand.

An adhesive strength between the attachment surface 24 of the vital sign monitoring sensor body 3 and the first connecting surface 16 of the connecting member 14 may be lower than the adhesive strength between a patient's skin and the second connecting surface 18 of the connecting member 14. Accordingly, as the operator lifts the vital sign monitoring sensor body 3 in a direction away from the patient's body, the connecting member 14 will remain attached to the patient's body. The vital sign monitoring sensor body 3 will then be free of any adhesives which facilitates future handling during e.g. re-cycling or re-conditioning. Further, it will cause less pain or discomfort to the patient when removing the connecting member 14 alone from the patient's skin, i.e. after having separated the vital sign monitoring sensor body 3 from the connecting member 14.

The adhesive strength between the attachment surface 24 of the vital sign monitoring sensor body 3 and the first connecting surface 16 of the connecting member 14 may be stronger than the adhesive strength between the supporting member 8 of the carrier 2 and the second connecting surface 18 of the connecting member 14. Accordingly, as the operator lifts the vital sign monitoring sensor body 3 from the carrier 2, the connecting member 14 will follow the sensor body 3.

At least the adhesive of the second connecting surface 18 should be provided by an adhesive allowed for medical use.

The adhesives used on the connecting member 14 could e.g. be a silicon-based adhesive or an acrylic-based adhesive.

Now turning to Fig. 5, an alternative embodiment of the connection between the sensor body 3 and a connecting member 14' is disclosed. This embodiment differs from the embodiment of Fig. 4 in that the connecting member 14' is configured to, instead of using an adhesive connection, mechanically connect to the attachment surface 24 of the vital sign monitoring sensor body 3. This may be made by at least one mechanical fastening member 50. The mechanical fastening member 50 may by way of example be a clip, a rivet or a screw. In the disclosed embodiment, three mechanical fastening members 50 in the form of rivets are highly schematically illustrated. The rivets are configured to extend through the connecting member 14' and into the sensor body 3. Hence, in this embodiment, the first connecting surface 16' of the connecting member 14' does not comprise any adhesive.

Referring to the embodiments of Figs. 3-5, the connecting member 14, 14' comprises a set of through-going cut-outs 31. The vital sign monitoring sensor body 3 may, on a surface thereof that is configured to face a patient's skin when the vital sign monitoring sensor body 3 is used on a patient, be provided with hydrogel. The hydrogel may be arranged on one or more isolated areas on the attachment surface 24 of the vital sign monitoring sensor body 3. By providing the connecting member 14 with corresponding cut-outs 31, the hydrogel is prevented from coming in contact with the connecting member 14, 14' and also with the carrier 2. The supporting members 8 of the carrier 2 may be provided with corresponding indents 33, see Fig. 1

The purpose of the hydrogel is to improve connectivity between the sensor body 3 and the patient's skin. To avoid the hydrogel from drying out over time, the aggregate may further comprise a moist carrier 43, such as a moist sponge, see Fig. 1. The protective sealing film 5 that is sealed to the carrier 2 and that is to be removed by the operator before use contributes to retain the humidity inside the aggregate until opening. The protective film 5 further ensures a sterile environment inside the thus sealed-off carrier.

As given above, it is preferred that the vital sign monitoring sensor body 3 with its communication module is passive as long as the aggregate and its contents is not to be used, which is the case during transportation and general handling/storing of the aggregate. This saves battery time and also reduces any noise signals.

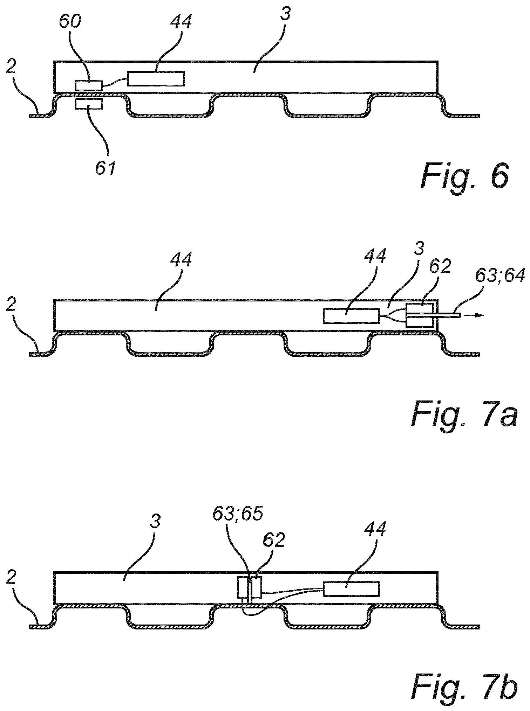

Turning to Fig. 6, a first embodiment of setting the vital sign monitoring sensor body 3 from a passive operating mode to an active operating mode is disclosed. According to this embodiment, the sensor body 3 comprises a magnetic sensor 60, such as a Reed switch supported by the PCB 44. Further, the carrier 2 supports a magnet 61. As long as the vital sign monitoring sensor body 3 is supported by the carrier 2, a magnetic field is formed between the magnetic sensor 60 and the magnet 61. When the sensor body 3 is removed from the carrier 2, the magnetic field is broken and hence the sensor body 3 is set from its passive operating mode to its active operating mode. This may be used to e.g. activate a radio module in the event the communication module comprises a radio module.

Turning to Fig. 7a, a second embodiment of setting the vital sign monitoring sensor body 3 from a passive operating mode to an active operating mode is disclosed. According to this embodiment, the vital sign monitoring sensor body 3 comprises an electrical switch 62 connected to the PCB 44. The electrical switch 62 is configured to be set from a passive operating mode to an active operating mode by removing an insulating member 63 being arranged in operational contact with the electrical switch 62 when the vital sign monitoring sensor body 3 is supported on the carrier 2.

The insulating member 63 may, as illustrated in Fig. 7a, be an insulating film 64 that is arranged in operational contact with the electrical switch 62, thereby breaking any communication across the electrical switch 62. To set the vital sign monitoring sensor body 3 from a passive operating mode to an active operating mode, the insulating member 63 must be actively removed, thereby allowing a communication across the electrical switch 62. The insulating film 64 may be a tab that is configured to be manually pulled, as is disclosed in Fig. 7a. Alternatively, the insulating film 64 may in one end be connected to the carrier 2. Thereby, the electrical switch may be configured to be automatically set from the passive operating mode to the active operating mode when removing the vital sign monitoring sensor body 3 from the carrier 2.

In another embodiment, see Fig. 7b, the insulating member 63 may be a protrusion 65 supported by the carrier 2. The insulating member 63 is arranged in operational contact with an electrical switch 62 in the sensor body 3. The insulating member 63 thereby breaks any communication across the electrical switch until the vital sign monitoring sensor body 3 is actively removed from the carrier 2. As the vital sign monitoring sensor body 3 is removed from the carrier 2, the electrical switch 62 is automatically set to an active operating mode.

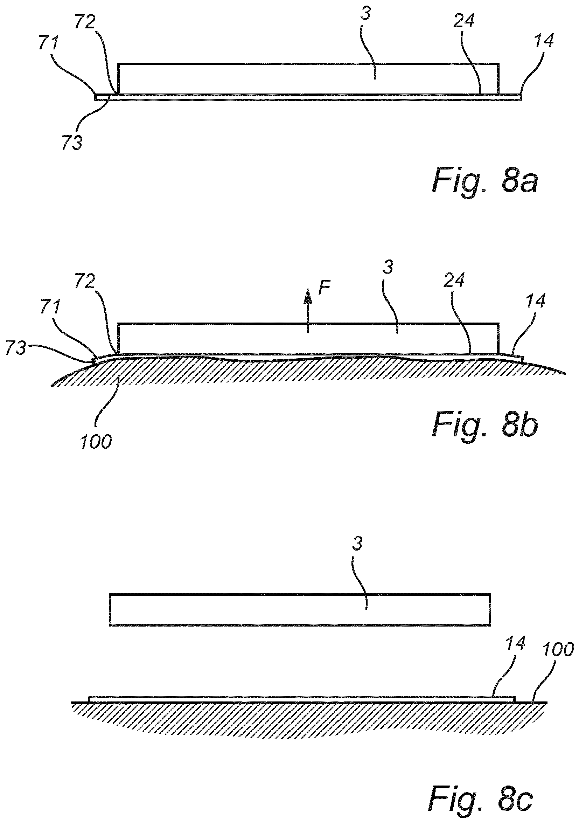

Fig. 8a discloses one embodiment where the outer boundary 71 of the connecting member 14 and the outer boundary 72 of the attachment surface 24 of the sensor body 3 both have the overall same cross-sectional geometry. A cross-sectional area delimited by the outer boundary 71 of the connecting member 14 is however larger than a cross-sectional area delimited by the outer boundary 72 of the attachment surface 24 of the sensor body 3. When interconnected, the connecting member 14 forms a skirt portion 73 that extends past the outer boundary 72 of the attachment surface 24 of the vital sign monitoring sensor body 3. By different material/physical configurations, the skirt portion 73 of the connecting member 14 is more flexible than the vital sign monitoring sensor body 3.

Now turning to Fig. 8b. This difference in flexibility increases the connecting strength between the patient's skin 100 and the connecting member 14 as seen in a condition when the sensor body 3 is mounted to the patient's body. Also, the skirt portion 73 of the connecting member 14 allows to better conform to the patient's body, which further increases the connecting strength. By the increased connecting strength between the connecting member 14 and the patient's skin 100, the sensor body 3 will, when pulled by a force F, separate from the connecting member 14 which in turn will remain adhered to the skin 100, see Fig. 8c. This allows the connecting member 14 to be peeled off separately from the skin in a less painful manner. Also, the recycling/reconditioning of the sensor body 3 is facilitated since it will be free from adhesive.

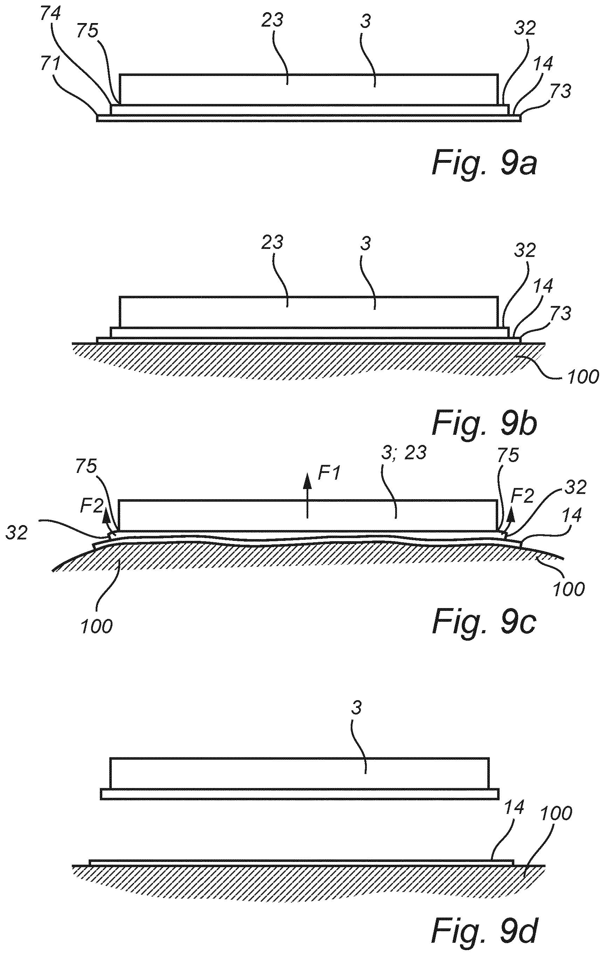

The very same effect can be achieved with the embodiment disclosed in Figs. 9a-9d, where the vital sign monitoring sensor body 3 comprises a flange portion 32, and where the connecting member 14 comprises a skirt portion 73 that extends past an outer boundary of the flange portion 32 of the vital sign monitoring sensor body 3. The outer boundary 71 of the connecting member 14 and the outer boundary 74 of the flange portion 32 of the sensor body 3 may both have the overall same cross-sectional geometry.

The flange portion 32 of the sensor body 3 is flexible. The flange portion 32 of the sensor body 3 should be flexible enough to allow the flange portion 32 to be pivoted in view of the housing 23 of the sensor body 3. The flange portion 32 of the sensor body 3 must not have a continuous extension along an outer periphery 75 of the sensor body 3. Further, the skirt portion 73 of the connecting member 14 may be more flexible than the flange portion 32 of the sensor body 3.

The flange portion 32 of the sensor body may be a member made of a different material than the housing 23 of the sensor body 3.

Fig. 9b discloses a state when the sensor body 3 is adhesively connected to the patient's skin 100 by means of the connecting member 14.

When removing the sensor body 3 from the patient's skin 100, see Fig. 9c, the operator pulls the sensor body 3 in the upward direction by a lifting force F1 while also lifting the flange portion 32 by a pivoting force F2. The lifting of the flange 32 will cause a pivoting of the flange 32 along the outer periphery 75 of the sensor body 3 which separates the sensor body 3 with its flange 32 from the connecting member 14. The connecting member 14 will remain adhered to the skin 100, see Fig. 9d. This allows the connecting member 14 to be peeled off separately from the skin in a less painful manner. The embodiment of Figs 9a-9d allows a very good and durable connection of the sensor body 3 to the patient's body, while at the same time being easy to remove.

In the following the overall handling of the aggregate 1 will be discussed by making reference to Fig. 1.

The operator opens the aggregate 1 by pulling off the protective sealing film 5, whereby the top area of the carrier 2 with its contents is freely exposed.
The operator grips a first vital sign monitoring sensor body 3 by one hand from above and pulls it by a lifting movement away from the carrier 2. During this movement, the adhesive engagement between the supporting member 8 of the carrier 2 and the adhesive layer 18a of the connecting member 14 is released, whereby the adhesive is freely exposed. As the sensor body 3 is removed from the carrier 2, it will, depending on its activation mode, be automatically set from a passive operation mode to an active operation mode. The operator maintains the grip of the vital sign monitoring sensor body 3 and presses it against the skin of the patient's body in a predetermined position. The vital sign monitoring sensor body 3 will adhesively attach to the skin by the adhesive layer 18a. This is repeated until all sensor bodies 3 have been attached to the patient's body. In case the sensor bodies 3 are pre-connected to one or several conducting members 4, the operator initiates the calibration of the sensor bodies 3. After calibration, the conducting members 4 may be disconnected from the sensor bodies 3 and the patient may move freely without being restricted by any wires while a wireless measurement may be prosecuted.

When the measurement is finished, the operator grips a sensor body 3 and pulls it away from the patient's body. Depending on the design of the connecting member 14, it may either remain adhesively attached to the persons skin, or follow the sensor body 3. The vital sign monitoring sensor body 3 may be disposed or collected for re-conditioning and re-use. In the event the connecting member 14 remains on the patient's body, it is removed by the operator pulling off the same by gripping its tab 20.

The person skilled in the art realizes that the present invention by no means is limited to the preferred embodiments described above. On the contrary, many modifications and variations are possible within the scope of the appended claims.

The wiring has been disclosed as a pre-connected wiring arranged to be pulled off from a support as the sensor body is lifted. It goes without saying that other types of wiring may be used, and also that the wiring even may be provided separate from the aggregate.

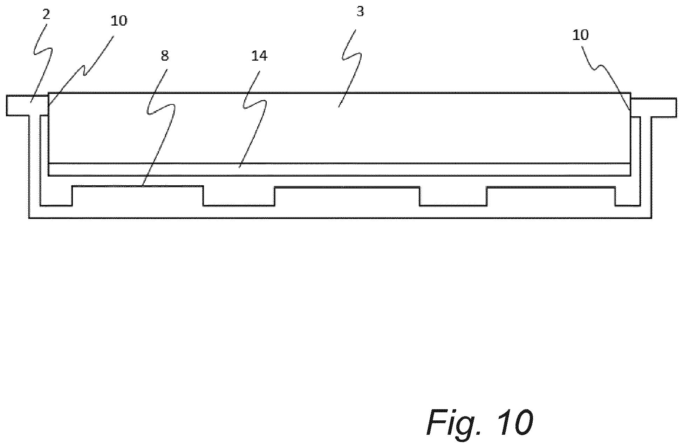

The sensor body has been described above as being supported on the carrier by the second connecting surface of the connecting member adhesively engaging the supporting member of the carrier. In an alternative embodiment, wall portions 10 adjacent the recesses 11 of the carrier 2 may contribute to mechanically, e.g. by friction, support the sensor body 3 in a position vertically above its respective supporting member 8. Thus, in such embodiment, there is no, or substantially no, adhesive contact between the supporting member 8 of the carrier 2 and the sensor body 3. This is best seen in Fig. 10.

It goes without saying that other identification means than a barcode may be used.

Variations to the disclosed embodiments can be understood and effected by the skilled person in practicing the claimed invention, from a study of the drawings, the disclosure, and the appended claims.

## Claims

1. An aggregate (1) comprising:
a vital sign monitoring sensor body (3) comprising at least one sensor (30);
a carrier (2) for supporting the vital sign monitoring sensor body (3), the carrier (2) comprising a supporting member (8; 10) configured to support the vital sign monitoring sensor body (3) on the carrier (2);
a connecting member (14; 14') comprising a first connecting surface (16a) and a thereto opposed second connecting surface (18a), wherein the first connecting surface (16a) connects to an attachment surface (24) of the vital sign monitoring sensor body (3) and the second connecting surface (18a) is configured to adhesively adhere to a patient's skin.

2. The aggregate according to claim 1, wherein upon removal of the vital sign monitoring sensor body (3) from the carrier (2), the vital sign monitoring sensor body (3) is arranged to be set from a passive operating mode to an active operating mode by breaking of a magnetic field being formed between a magnetic sensor (60) of the vital sign monitoring sensor body (3) and a magnet (61) of the carrier (2), said magnetic field being formed as long as the vital sign monitoring sensor body (3) is supported by the carrier (2).

3. The aggregate according to claim 1, wherein the vital sign monitoring sensor body (3) comprises an electrical switch (62), and wherein the vital sign monitoring sensor body (3)is configured to be set from a passive operating mode to an active operating mode by removing an insulating member (63; 64; 65) being arranged in operational contact with the electrical switch (62) in a condition when the vital sign monitoring sensor body (3) is supported on the carrier (2).

4. The aggregate according to claim 1, wherein the vital sign monitoring sensor body (3) is mechanically and releasably supported by the supporting member (8; 10) of the carrier (2), and wherein in a condition when the vital sign monitoring sensor body (3) is mechanically supported by the carrier (2), there is no, or substantially no, adhesive contact between the supporting member (8; 10) of the carrier (2) and the second connecting surface (18a) of the connecting member (14).

5. The aggregate according to claim 1, wherein the first connecting surface (16a) mechanically connects the connecting member (14') to the attachment surface (24) of the vital sign monitoring sensor body (3) by at least one mechanical fastening member (50).

6. The aggregate according to claim 1, wherein the first connecting surface (16a) adhesively connects the connecting member (14) to the attachment surface (24) of the vital sign monitoring sensor body (3) by a releasable adhesive or by a permanent adhesive.

7. The aggregate according to claim 1, wherein the vital sign monitoring sensor body (3) is adhesively and releasably supported by the supporting member (8) of the carrier (2) by an adhesive of the second connecting member (18a).

8. The aggregate according to claim 7, wherein at least a portion of the supporting member (8) of the carrier (2) is coated with an easy release coating material.

9. The aggregate according to any of claims 7 or 8, wherein a contact area formed between the supporting member (8) of the carrier (2) and the second connecting surface (18a) of the connecting member (14; 14') is smaller than a total cross sectional area of the second connecting surface (18a).

10. The aggregate according to any preceding claims, wherein an adhesive strength between the attachment surface (24) of the vital sign monitoring sensor body (3) and the first connecting surface (16) of the connecting member (14) is lower than the adhesive strength between a patient's skin and the second connecting surface (18a) of the connecting member (14).

11. The aggregate according to any of the preceding claims, wherein the connecting member (14) comprises a skirt portion (73) extending past an outer boundary of the attachment surface (24) of the vital sign monitoring sensor body (3), the skirt portion (73) of the connecting member (14) being more flexible than the vital sign monitoring sensor body (3).

12. The aggregate according to any of claims 1-10, wherein the vital sign monitoring sensor body (3) comprises a flange portion (32), and wherein the connecting member (14) comprises a skirt portion (73) extending past an outer boundary (72) of the flange portion (32) of the vital sign monitoring sensor body (3), wherein the skirt portion (73) of the connecting member (14) is more flexible than the flange portion (32) of the vital sign monitoring sensor body (3).

13. The aggregate according to any of the preceding claims, wherein the vital sign monitoring sensor body (3) comprises one or more sensors (30) from the group consisting of an ECG (Electrocardiography) sensor, a pulse oximeter sensor, a blood pressure sensor, a temperature sensor, a glucose sensor, a cortisol sensor, an oxygen sensor, a carbon dioxide sensor and a respiration sensor.

14. The aggregate according to any of the preceding claims, wherein the aggregate (1) comprises two or more vital sign monitoring sensor bodies (3), and wherein the two or more sensor bodies (3) are connected by one or more electrically conducting members (4) while being supported in the carrier (2), and wherein
the connection with the one or more electrically conducting members (4) is configured to remain after removal of the two or more vital sign monitoring sensor bodies (3) from the carrier (2); or
wherein the one or more electrically conducting members (4) is/are configured to be disconnected after removal of the two or more vital sign monitoring sensor bodies (3) from the carrier (2).

15. The aggregate according to claim 14, wherein the electrically conductive members (4) are formed by a laminate comprising a flexible film supporting one or more flexible insulators and one or more flexible metal films.

16. The aggregate according to any of the preceding claims, wherein the aggregate (2) comprises an electrical connector (40) arranged in direct or indirect communication with the vital sign monitoring sensor body (3), said electrical connector (40) allowing the vital sign monitoring sensor body (3) to be charged when being supported by the carrier (2).
